# EUROPEAN PATENT SPECIFICATION

(11) **EP 2 640 686 B1**
(45) Date of publication and mention of the grant of the patent: **24.12.2014**
(21) Application number: 11791611.4
(22) Date of filing: 18.11.2011
(51) Int. Cl.: C07C 51/31

(54) **NITROUS OXIDE-CONTAINING IONIC LIQUIDS AS CHEMICAL REAGENTS**
STICKOXIDHALTIGE IONISCHE FLÜSSIGKEITEN ALS CHEMISCHE REAGENZIEN
OXYDE DE DIAZOTE-CONTENANT DES LIQUIDES IONIQUES EN TANT QUE RÉACTIFS CHIMIQUES

(30) Priority: 19.11.2010 GB 201019701
(43) Date of publication of application: 25.09.2013
(73) Proprietor: Invista Technologies S.A R.L., 9000 St. Gallen (CH)
(72) Inventor: AKI, Sudhir, CH-9000 St. Gallen (CH); WHISTON, Keith, CH-9000 St. Gallen (CH); BELHOCINE, Tayeb, Belfast BT7 1NN (GB); SEDDON, Kenneth, Richard, Belfast BT7 1NN (GB)
(74) Representative: Cockerton, Bruce Roger
(86) International application number: PCT/GB2011/001628
(87) International publication number: WO 2012/066296

(56) References cited:
- WO-A1-2010/094891
- GB-A- 2 427 192
- KIMURA A ET AL: "Study on the reaction of chlorophenols in room temperature ionic liquids with ionizing radiation", RADIATION PHYSICS AND CHEMISTRY, ELSEVIER SCIENCE PUBLISHERS BV., AMSTERDAM, NL, vol. 77, no. 10-12, 1 October 2008 (2008-10-01), pages 1253-1257, XP025407539, ISSN: 0969-806X, DOI: 10.1016/J.RADPHYSCHEM.2008.05.020 [retrieved on 2008-05-23]
- DATABASE WPI Week 200874 Thomson Scientific, London, GB; AN 2008-M51687 XP002676385, & CN 101 252 027 A (CHINA ATOM ENERGY SCI RES INST) 27 August 2008 (2008-08-27)
- ANNE-LAURE REVELLI ET AL: "Reducing of Nitrous Oxide Emissions Using Ionic Liquids", THE JOURNAL OF PHYSICAL CHEMISTRY B, vol. 114, no. 24, 24 June 2010 (2010-06-24), pages 8199-8206, XP55027366, ISSN: 1520-6106, DOI: 10.1021/jp103734c
- GUI JIANZHOU ET AL: "Clean synthesis of adipic acid by direct oxidation of cyclohexene with H2O2 catalysed by Na2WO4.2H2O and acidic ionic liquids", JOURNAL OF CHEMICAL RESEARCH, SCIENCE REVIEWS LTD, GB, vol. 8, 1 August 2005 (2005-08-01), pages 520-522, XP009159393, ISSN: 0308-2342, DOI: 10.3184/030823405774663363
- A SHIMIZU ET AL: "Abatement technologies for N2O emissions in the adipic acid industry", CHEMOSPHERE - GLOBAL CHANGE SCIENCE, vol. 2, no. 3-4, 1 July 2000 (2000-07-01), pages 425-434, XP55027782, ISSN: 1465-9972, DOI: 10.1016/S1465-9972(00)00024-6
- G. CENTI ET AL: "Removal of N 2 O from Industrial Gaseous Streams by Selective Adsorption over Metal-Exchanged Zeolites", INDUSTRIAL & ENGINEERING CHEMISTRY RESEARCH, vol. 39, no. 1, 1 January 2000 (2000-01-01), pages 131-137, XP55027487, ISSN: 0888-5885, DOI: 10.1021/ie990360z

## Description

The invention relates to the use of an N₂O-containing ionic liquid as a reagent in a chemical reaction, namely as an oxidising agent. A reaction process in which ionic liquids are used to absorb nitrous oxide (hereinafter referred to as N₂O) from a mixture of by-products produced during the reaction process and a process for the synthesis of adipic acid using nitric acid which employs an ionic liquid to absorb N₂O from a mixture of by-products produced during the synthesis are also disclosed.

It is known that N₂O can be used as an oxidising agent. For example, the oxidation of an olefinic compound to an aldehyde or a ketone by means of N₂O is described in GB 649,680 or the equivalent U.S. Pat. No. 2,636,898. Both documents quite generally disclose that the oxidation can in principle be effected in the presence of a suitable oxidation catalyst.

G. L. Panov et al., "Non-Catalytic Liquid Phase Oxidation of Alkenes with Nitrous Oxide. 1. Oxidation of Cyclohexene to Cyclohexanone", React. Kinet. Catal. Lett. Vol. 76, No. 2 (2002) p. 401-405; and K. A. Dubkov et al., "Non-Catalytic Liquid Phase Oxidation of Alkenes with Nitrous Oxide. 2. Oxidation of Cyclopentene to Cyclopentanone", React. Kinet. Catal. Lett. Vol. 77, No. 1 (2002) p. 197-205 likewise describe oxidations of olefinic compounds with N₂O. "Liquid Phase Oxidation of Alkenes with Nitrous Oxide to Carbonyl Compounds" by E. V. Starokon et al. in Adv. Synth. Catal. 2004, 346, 268-274 also includes a mechanistic study of the oxidation of alkenes with N₂O in the liquid phase.

The synthesis of carbonyl compounds from alkenes with N₂O is also described in various international patent applications. For instance, WO 03/078370 discloses a process for preparing carbonyl compounds from aliphatic alkenes with N₂O. The reaction is carried out at temperatures in the range from 20 to 350°C and pressures of from 0.01 to 100 atm. WO 03/078374 discloses a corresponding process for preparing cyclohexanone. According to WO 03/078372, cyclic ketones having from 4 to 5 carbon atoms are prepared. According to WO 03/078375, cyclic ketones are prepared under these process conditions from cyclic alkenes having from 7 to 20 carbon atoms. WO 03/078371 discloses a process for preparing substituted ketones from substituted alkenes. WO 04/000777 discloses a process for reacting di- and polyalkenes with N₂O to give the corresponding carbonyl compounds.

A more recent study of the use of N₂O as an oxidising agent can be found in V.N. Parmon et al "Nitrous oxide in oxidation chemistry and catalysis: application and production", Catalysis Today, 100 (2005), 115-131.

Various preparation processes for N₂O are known in the art. For example, WO 98/25698 discloses a process for preparing N₂O by catalytic partial oxidation of NH₃ with oxygen. According to WO 98/25698, a catalyst composed of manganese oxide, bismuth oxide and aluminium oxide is used, which leads to N₂O with high selectivity. A similar catalyst system is also described in detail in a scientific study (Noskov et al., Chem. Eng. J. 91 (2003) 235-242). U.S. Pat. No. 5,849,257 likewise discloses a process for preparing N₂O by oxidation of ammonia. The oxidation takes place in the presence of a copper-manganese oxide catalyst. WO 00/01654 discloses a process in which N₂O is prepared by reducing a gas stream comprising NOₓ and ammonia.

N₂O is also obtained as a by-product in various chemical processes, especially in oxidations with nitric acid. For example, in industry, the synthesis of adipic acid is generally carried out via the nitric acid oxidation of cyclohexanol, cyclohexanone or a mixture of the two (commonly referred to as ketone-alcohol oil or KA oil). N₂O is the major by-product in the synthesis, along with other nitrogen oxides, carbon dioxide, and some lower molecular mass dicarboxylic acids. Some by-products arising from impurities in the KA oil are also present.

Other examples of processes in which N₂O is obtained as a by-product are the oxidation of cyclododecanone and/or cyclododecanol with nitric acid to give dodecanedicarboxylic acid and the partial oxidation of NH₃ to NO.

The formation of N₂O in these processes presents a number of problems for industry. For example, as the recovery and purification processes are generally not cost effective, the usual practice is to abate the N₂O as waste gas and no commercial value is derived from it. This has consequences because N₂O is a strong greenhouse gas (at least 100 times the strength of CO₂) and industry is coming under increasing pressure to reduce the emission of greenhouse gases into the atmosphere. Therefore, it would be advantageous if N₂O produced in industrial processes could be captured and used in an economical and efficient way, for example as an oxidising agent in a subsequent chemical reaction, rather than simply being abated as a waste gas.

US 2008/0274032 describes a process for purifying and concentrating N₂O containing off-gases obtained from various chemical processes. However, the process requires a number of successive absorption and desorption steps and uses a combination of organic solvents and water, to produce the N₂O solutions. Thus, the recovery of the purified N₂O requires extensive and expensive downstream refining steps. Furthermore, it is not contemplated that the N₂O solutions obtained by the processes can be used directly in a further reaction process.

A.-L. Revelli et al., "Reducing of Nitrous Oxide Emissions Using Ionic Liquids", J. Phys. Chem. B Vol.114 (2010) p.8199-8206 discloses the ability of ionic liquids to absorb N₂O from emissions.

WO 2010/094891 discloses a treatment process of kitchen utensils including an oxidation step, wherein the oxidation step may be carried out in ionic liquid media. N₂O is disclosed as a suitable oxidising agent for the oxidation step.

The inventors have found that the use of an ionic liquid enables N₂O produced in industrial processes to be readily and selectively absorbed and thus captured for later use, so avoiding unfavourable abatement of gaseous N₂O into the atmosphere. The N₂O-containing ionic liquid thus obtained has particularly advantageous properties such as thermal stability and low vapour pressure which enables the N₂O to be recovered relatively easily, if desired. Ionic liquids are also good solvents for a range of other substrates, and this enables the N₂O-containing ionic liquids obtained to be used directly as oxidising agents in further chemical processes.

Moreover, N₂O-containing ionic liquids as oxidising agents have been found to give favourable conversion of reactants to products, and to lead to higher selectively for the desired product than corresponding reactions in the absence of an ionic liquid. Thus, the use of N₂O-containing ionic liquids gives higher yields and higher conversion to the desired reaction products.

The invention provides the use of an N₂O-containing ionic liquid as a reagent in a chemical reaction as an oxidising agent according to claim 1. The N₂O-containing ionic liquid can be used as a reagent in a number of different reactions, for example as an epoxidising agent, in oxidative dehydrogenation reactions and as a catalyst.

In one aspect, the invention provides the use of an N₂O-containing ionic liquid as a reagent in a chemical reaction for the purpose of improving the reaction yield and conversion compared to a reaction conducted in the absence of an ionic liquid. In another aspect, the invention provides the use of an N₂O-containing ionic liquid as a reagent in a chemical reaction for the purpose of improving the selectivity of the reaction for the desired product compared to a reaction conducted in the absence of an ionic liquid.

In other aspects, the invention also provides a method corresponding to the use described herein. Thus, the invention provides a method of conducting a chemical reaction, the method comprising contacting one or more reactants with an N₂O-containing ionic liquid.

As used herein, an N₂O-containing ionic liquid describes a composition in which an ionic liquid and N₂O are associated with each other. For example, in one embodiment the N₂O-containing ionic liquid is a solution of N₂O in an ionic liquid. However, the N₂O-containing ionic liquids of the invention are not limited to such physical solvent-solute interactions between the ionic liquid and the N₂O and they also encompass relationships in which there is some chemical interaction between the N₂O and the ionic liquid, for example the formation of a lewis acid/base adduct, or the chemical reaction of the N₂O and the ionic liquid to form N₂O and ionic liquid derivatives which themselves are associated with each other for example the formation of an intermediate peroxide or epoxide compound from reaction of the N₂O.

The N₂O-containing ionic liquid is used as an oxidising agent. The N₂O-containing ionic liquid is obtained via the reaction process described in claim 1.

The N₂O-containing ionic liquid can be used as an oxidising agent in a number of different oxidation reactions. For example, in one aspect, the use or method comprises oxidising benzene to produce phenol or oxidising cyclohexene to cyclohexanol, cyclohexanone or a mixture of cyclohexanol and cyclohexanone. In another aspect the use or method comprises the oxidative dehydrogenation of an alcohol to a ketone or the oxidative dehydrogenation of butene to 1,3-butadiene.

In one aspect, the N₂O-containing ionic liquid is used as an epoxidising agent. For example, the N₂O-containing ionic liquid is reacted with an olefin to produce an epoxide.

In another aspect, the use of the N₂O-containing ionic liquid, or the method of conducting a chemical reaction, comprises the oxidation of cyclopentene to cyclopentanone.

In yet another aspect, the use of the N₂O-containing ionic liquid, or the method of conducting a chemical reaction, comprises the oxidation of cyclododecene (CDDC) to cyclododecanone (CDDK).

Ionic liquids or low temperature molten salts are known in the art as very low volatility solvents suitable for carrying out a range of chemical processes. For example, the solubility of N₂O in certain ionic liquids has been investigated *("*Anion Effects on Gas Solubility in Ionic Liquids ", J.Phys.Chem.B 2005, 109, 6366-6374, Brennecke *et al.)*

However it has not previously been disclosed that ionic liquids can be employed as selective solvents for absorption of N₂O from a mixture of by-products produced in an oxidation process, particularly an adipic acid synthesis process. Furthermore, the use of an N₂O-containing ionic liquid as a reagent in a chemical reaction has not been investigated previously.

An ionic liquid is a liquid that contains essentially only ions, i.e. molten salts, although some ionic liquids are in a dynamic equilibrium wherein the majority of the liquid is made up of ionic rather than molecular species. In the art, the term "ionic liquid" is used to refer to such salts whose melting point is below 100°C. Such ionic liquids are sometimes also referred to as room temperature ionic liquids (RTIL). Ionic liquids are typically salts of bulky and asymmetric organic cations. For instance, US 7,157,588 teaches compositions based on N-substituted pyrrolidinones having a pendant ammonium cation separated from the pyrrolidone ring by a variable length alkyl spacer. WO 2006/136529 teaches pyrazolium alkylsulfates and a method for their production. WO 2008/150842 describes a broad class of ionic liquids comprising heterocyclic nitrogen containing cations.

The term "ionic liquid" as used in the context of the invention refers to a salt whose melting point is below about 100°C. Optionally, the ionic liquids used in the invention comprise an organic cation. Organic cations are cations containing at least one carbon atom. Typically, the organic cation contains at least one alkyl group. Conveniently, the ionic liquid comprises an asymmetric organic nitrogen containing cation. In one aspect, the ionic liquids used in the invention comprise an organic anion. Organic anions are anions containing at least one carbon atom. Typically, organic anions are anions containing at least one carbon atom but excluding carbonate anions or bicarbonate anions. In another aspect, the ionic liquids used in the invention comprise an organic cation and an organic anion, wherein the organic cation and anion are as defined above. In yet another aspect, the ionic liquids used in the invention comprise an inorganic anion.

The use and method of the present invention may utilise a single ionic liquid or a mixture of two or more ionic liquids. Typically, one or two, and typically only one ionic liquid is used.

Thus in one embodiment the ionic liquid comprises a cation selected from one or more of 1-alkylpyridinium (N-alkylpyridinium); alkyl- or polyalkyl-pyridinium; alkyl- or polyalkyl-guanidinium (particularly tetraalkylguanidinium); phosphonium (PR₄⁺); alkyl- or polyalkyl-phosphonium (particularly tetraalkylphosphonium); imidazolium, alkyl- or polyalkyl-imidazolium (particularly 1,3-dialkylimidazolium); ammonium (NR₄⁺), alkyl- or polyalkyl-ammonium (particularly tetraalkylammonium); alkyl- or polyalkyl-pyrazoliuml; alkyl- or polyalkyl-pyrrolidinium (particularly dialkylpyrrolidinium); alkyl- or polyalkyl-piperidinium (particularly 3-methylpiperidinium); alkyl- or polyalkylazepanium; alkyl or polyalkyl-azepinium, alkyloxonium, and alkysulfonium. Each R group of the phosphonium and ammonium cations may be independently selected from hydrogen, hydroxyl, alkyl, alkyl ethers, alkyl esters, alkyl amides, alkyl carboxylic acids, or sulfonate. Examples include N-ethylpyridinium; N-methyl-N-alkylpyrrolidinium such as N-butyl-N-methylpyrrolidinium; N-methyl-N-(butyl-4-sulfonic acid)pyrrolidinium; 1-alkyl-3-alkylimidazolium such as 1-butyl-3-methylimidazolium (bmim) and N-methyl-N'-ethylimidazolium; trimethyl-(2-hydroxyethyl)ammonium; and tetradecyltrihexylphosphonium ([CAS# 258864-54-9], referred to herein as [P_{66614]}).

A number of different anions may be employed, including inorganic anions and large organic anions. In one embodiment, the anion of the ionic liquid(s) is selected from one or more of a halide (conveniently chloride, bromide or iodide), nitrate, an alkylsulfonate or alkyl polyalkoxysulfonate, hydrogensulfonate, hexafluorophosphate and tetrafluoroborate, and other anions based on nitrogen, phosphorous, sulphur, boron, silicon, selenium, tellurium, gallium, indium, halogens, and oxoanions of metals. Suitable anions include, but are not limited to tetrafluoroborate (BF₄⁻), bis(trifluoromethylsulfonyl)imide (NTf₂⁻), hydrogensulfate (HSO₄⁻), methylsulfonate, trifluoromethylsulfonate, methoxyethylsulfonate, 2-methoxyethylsulfonate, ethoxyethylsulfonate, 2-ethoxyethylsulfonate, (methoxypropoxy)propylsulfonate, 1-(1-methoxypropoxy)-propylsulfonate, (methoxyethoxy)-ethylsulfonate, 1-(1-methoxyethoxy)-ethylsulfonate, methyl(diethoxy)ethylsulfonate, 1-methyl(diethoxy)ethylsulfonate, toluene-4-sulfonate, trifluoromethylsulfonyl, carboxylate, formate, acetate, dicyanimide, trifluoroacetate, bis(trifluoromethanesulfonyl)imide, [Ga₂Cl₇]⁻, [InCl₄]⁻ and [In₂Cl₇]⁻.

When a mixture of two or more ionic liquids is used, the cation and/or the anion of each of the ionic liquids present in the mixture may be the same or different. In one embodiment, the or each ionic liquid comprises at least one C₂ - C₆ alkyl group. The C₂ - C₆ alkyl group may be a substituent on either the anion or the cation of the ionic liquid(s). Optionally, the C₂ - C₆ alkyl group is a substituent on the cation of the ionic liquid(s).

In one embodiment, the ionic liquid is selected from; 1-n-butyl-3-methylimidazolium[Ga₂Cl₇], 1-n-butyl-3-methylimidazolium[In₂Cl₇], 1-n-butyl-3-methylimidazolium bis(trifluoromethylsulfonyl)imide, tetradecyltrihexylphosphonium[Ga₂Cl₇] tetradecyltrihexylphosphonium [InCl₄] and tetradecyltrihexylphosphonium bis(trifluoromethylsulfonyl)imide.

The invention also provides a reaction process comprising the steps, (a) contacting a reactant with a reagent to produce a product and a mixture of by-products comprising N₂O; and (b) contacting the mixture of by-products with an ionic liquid to absorb the N₂O to produce an N₂O-containing ionic liquid.

The reaction process can be any one in which N₂O is produced. Thus, the reagent can be an oxidising agent, a reducing agent, a catalyst, a nucleophile or an electrophile. In one aspect, the reaction is an oxidation reaction, the reagent is an oxidising agent and the product is an oxidised product.

Thus, N₂O present in a mixture of by-products produced in a reaction process, for example an oxidation process, can be absorbed, for example selectively, into an ionic liquid to form an N₂O-containing ionic liquid. This process therefore enables the N₂O to be recovered and used, rather than it being abated into the atmosphere.

In one aspect, the invention provides an oxidation process as described above, wherein the oxidising agent is a nitrogen containing oxidising agent. For example, the oxidising agent is N₂O or nitric acid. Typically, the oxidising agent is nitric acid.

In the aspect of the invention where the oxidising agent is N₂O, the N₂O present in the mixture of by-products represents the portion of the N₂O used as the oxidising agent which is not consumed during the oxidation reaction. Thus, in this embodiment, the unreacted N₂O can be recovered and reused in another oxidation process, or recycled back into the same oxidation process.

In one aspect, the invention provides an oxidation process comprising steps (a) and (b) as described above, wherein the reactant is selected from cyclohexanol, cyclohexanone or a mixture of cyclohexanol and cyclohexanone. In one embodiment the oxidised product formed in step (a) is adipic acid. In a preferred embodiment, the oxidising agent is nitric acid, the reactant is selected from cyclohexanol, cyclohexanone or a mixture of cyclohexanol and cyclohexanone and the oxidised product is adipic acid. Thus, the preferred oxidation process of the invention represents the synthesis of adipic acid, in which a mixture of by-products comprising N₂O is produced, the mixture of by-products being contacted with an ionic liquid to absorb the N₂O to produce an N₂O-containing ionic liquid. Thus, in this embodiment, the synthesis of adipic acid is made more economical and environmentally friendly, as the by-product N₂O is recovered for further use and not abated into the atmosphere.

Differences among commercial adipic acid synthesis processes are mainly in the manufacture of the KA oil. In most processes the six carbon atoms of the adipic acid backbone come from benzene. In one process benzene is hydrogenated to cyclohexane and the cyclohexane is oxidised to give the KA oil. In an alternative process, benzene is first oxidised to phenol, the phenol then being hydrogenated to give the KA oil.

Thus, in one aspect of the oxidation process of the invention, the reactant is produced by the oxidation of cyclohexane and the cyclohexane is produced by the hydrogenation of benzene.

In another aspect of the oxidation process of the invention, the reactant is produced by the hydrogenation of phenol and the phenol is produced by the oxidation of benzene.

Adipic acid is a commercially important aliphatic dicarboxylic acid. Its primary application is in the production of nylon 6,6. Thus, in one aspect, the invention provides a process for the manufacture of nylon 6,6 from the adipic acid produced via the oxidation process described above. Therefore, the process of the invention allows for a more economical and environmentally friendly synthesis of nylon, owing to the recovery and re-use of the N₂O produced in synthesis of the intermediate adipic acid.

In one aspect, in addition to N₂O, the mixture of by-products obtained in step (a) of the process of the invention contains one or more components selected from; oxides of carbon (for example, carbon monoxide and carbon dioxide), nitrogen, oxides of nitrogen (for example, NO, NO₂, N₂O₃, N₂O₄ and N₂O₅ - known collectively as NOx) and C₁-C₅ dicarboxylic acids. The N₂O is separated from the other components of the mixture of by-products owing to the selective absorption of the N₂O into the ionic liquid to form the N₂O-containing ionic liquid. The solubility of NOx in ionic liquids is very low (see J. Phys. Chem. B 2007, 111, 7778-7785) which gives a solubility of 2.5g/Kg of EMIM NTf₂ compared with 50 to 100 g/Kg for N₂0. Carbon monoxide and nitrogen are very insoluble in typical ionic liquids (see J. Phys. Chem. B 2005, 109, 6366-6374 in which the authors report undetectable solubility for carbon monoxide in ionic liquids). Carbon Dioxide has a solubility similar to N₂O in Ionic Liquids, but its presence as an inert in the oxidant system is not a problem for the invention.

In one embodiment, the oxidation process of the invention further comprises a step (c) of using the N₂O-containing ionic liquid produced in step (b) as an oxidising agent. Optionally, the use of the N₂O-containing ionic liquid comprises the oxidation of benzene to produce phenol.

In one aspect, the N₂O-containing ionic liquid produced in step (b) is recycled and used as an oxidising agent to oxidise benzene to phenol, said phenol being subsequently hydrogenated to produce the reactant used in step (a) of the process of the invention. Thus, in this aspect, the by-product N₂O is recovered and recycled back into the process.

For example, in the case of the synthesis of adipic acid described above, starting material benzene is oxidised to produce phenol, and the phenol is hydrogenated to produce the KA oil. The KA oil is then contacted with nitric acid, according to the embodiment described above, to produce adipic acid and a mixture of by-products containing N₂O. The mixture of by-products containing N₂O is contacted with an ionic liquid to absorb the N₂O to produce an N₂O-containing ionic liquid. The N₂O-containing ionic liquid thus obtained is then recycled back into the synthesis and used as an oxidising agent in the oxidation of starting material benzene to produce phenol. Thus, the invention greatly increases the economy of the process, as N₂O, which is conventionally considered to be a waste by-product, can now be used as an oxidising agent in the synthesis.

In one aspect, the invention provides an oxidation process described above, further comprising the step of recovering the N₂O from the N₂O-containing ionic liquid by desorption of the N₂O. For example, the N₂O is recovered via thermal desorption or pressure swing desorption. Conveniently, the N₂O is recovered via thermal desorption. The ionic liquids used in the present invention are suitable for this process owing to their high capacity for the reversible absorption of N₂O. That is, N₂O is readily absorbed in the ionic liquids but it is also readily desorbed by heating or lowering the pressure. Typically, the temperature of the desorption step is at or below 200°C. Preferably, the temperature is at or below 150°C, more preferably at or below 100°C, more preferably, at or below 50°C.

Typical saturation solubilities for the N₂O-containing ionic liquids of the present invention at room temperature are approximately 0.3, 0.4, 0.5, 0.6, 0.7, 0.8 or 0.9 mol% for a Henry's constant of 30. At higher pressure and lower temperature solubilities above 1 mol% may be achieved. More preferably, solubilities above 5 mol%, more preferably above 10 mol %, more preferably above 15 mol% can be achieved.

In certain embodiments the N₂O-containing ionic liquid is obtained from a commercial adipic acid synthesis. Such adipic acid syntheses are described by M.T Musser, Ullmann's Encyclopedia of Chemical Technology, 5th edition, Al, 259, incorporated herein by reference. Adipic acid synthesis results in the production of N₂O as a by-product. Accordingly, contacting the N₂O with an ionic liquid enables an N₂O-containing ionic liquid to be obtained as a useful additional product form the adipic acid synthesis.

In a further embodiment the N₂O can be obtained as a by-product in the off gas from a nitric acid production process. In a still further embodiment the N₂O can be produced as a by-product of a hydroxylamine production process.

The invention is illustrated by the non-limiting embodiments described below, in which the following figures are referred to:
**Figure 1** shows the GC analysis of the reaction mixture for the reaction of CDDC with N₂O at 30bar, 200°C for 12 hours, in the absence of an ionic liquid.
**Figure 2** shows the GC analysis of the organic layer of the reaction mixture for the reaction of CDDC with N₂O-containing [bmim][NTf₂] ionic liquid, at 30bar, 200°C for 12 hours.
**Figure 3** shows the ¹H NMR analysis of the ionic liquid layer of the reaction mixture for the reaction of CDDC with N₂O-containing [bmim][NTf₂] ionic liquid, at 30bar, 200°C for 12 hours.
**Figure 4** shows ¹H NMR analysis of the ionic liquid layer of the reaction mixture for the reaction of CDDC with N₂O-containing [P₆₆₆₁₄][NTf₂] ionic liquid, at 30bar, 200°C for 12hours.

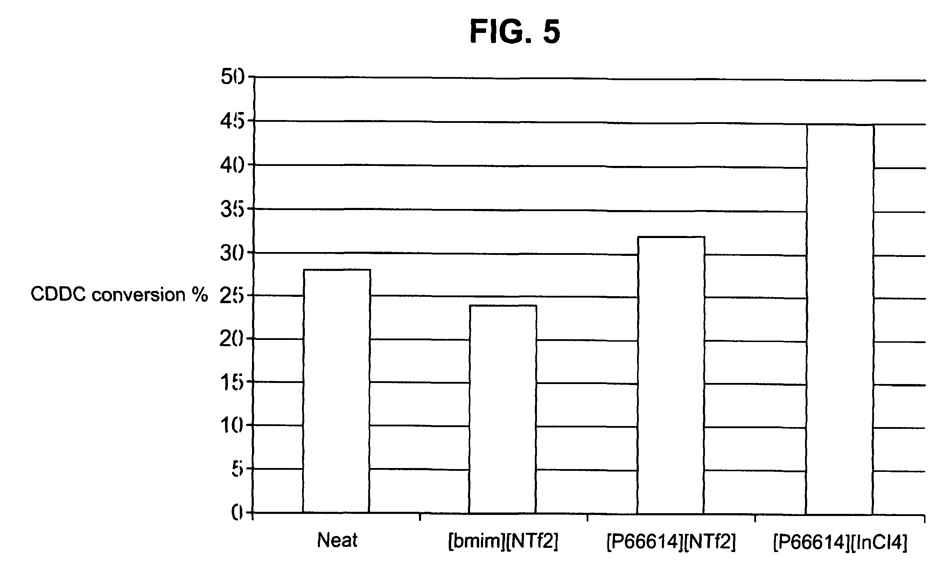

**Figure 5** shows a summary of % CDDC conversion for reaction of CDDC with N₂O and various N₂O-containing ionic liquids at 200°C, 45bar for 12 hours.
**Figure 6** shows a GC calibration curve for CDDC.
**Figure 7** shows a GC calibration curve for CDDK.

### Experimental

### Materials

Cyclododecene (CDDC) (mixture *cis*/*trans*) was purchased from sigma-Aldrich. Analysis by ¹H NMR and GC-MS show that the mixture content is 25.5 % *cis* and 71.6 % *trans.* [bmim][NTf₂] was synthesised in the laboratory. [bmim][Ga₂Cl₇] was prepared by reacting [bmim]Cl with GaCl₃ (2 eq.). [P₆₆₆₁₄]Ga₂Cl₇ was made from the corresponding chloride [P₆₆₆₁₄]Cl using GaCl₃ in the laboratory. [P₆₆₆₁₄]InCl₄ was made from the corresponding chloride [P₆₆₆₁₄]Cl using InCl₃ in the laboratory. [P₆₆₆₁₄]NTf₂ was made in the laboratory from [P₆₆₆₁₄]Cl. Nitrous oxide was purchased from Air liquide.

### Neat reactions

### General procedure

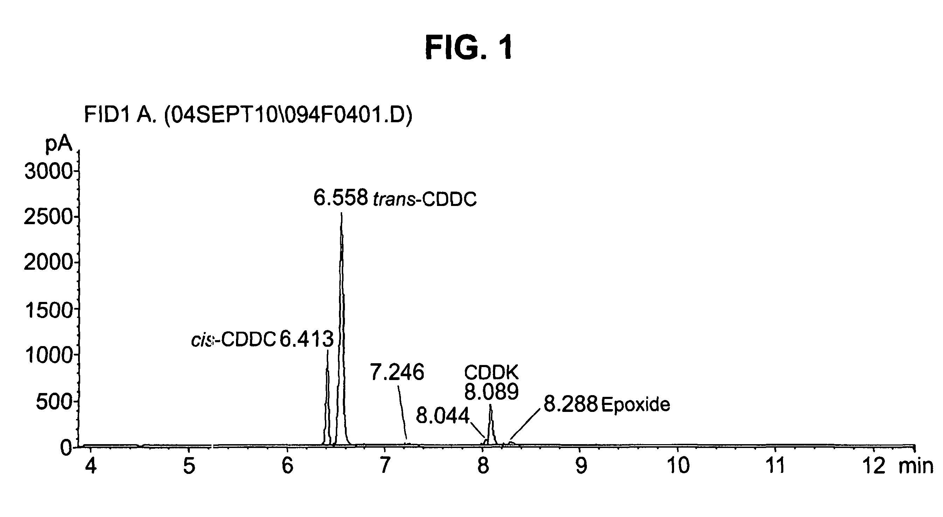

A 20 cm³ autoclave was initially charged with 1 g of cyclododecene (6.01mmol), sealed and flushed with argon. Subsequently, N₂O (10 to 45 bar) was injected to the autoclave and the temperature increased to 200 °C. After 12 h reaction, the autoclave was cooled and decompressed. After dilution in cyclohexane, a sample was analysed by means of quantitative GC (see **Figure 1**). The different results are summarised in Table 1.

**Table 1**

| Pressure N₂O injected (bar) at 20 °C | Temperature (°C) | Maximum pressure in the autoclave | Time (h) | Conversion (%) | CDDK-selectivity (%) |
|---|---|---|---|---|---|
| 10 | 200 | 16 | 12 | 8 | ≥ 99 |
| 20 | 200 | 38 | 12 | 15 | 98* |
| 30 | 200 | 53 | 12 | 18 | 96* |
| 45 | 200 | 78 | 12 | 28 | 93* |

| | | | | | |
|---|---|---|---|---|---|
| *formation of epoxide and other products was also observed by GC-MS | | | | | |

### Reaction with N₂O-containing ionic liquid

### General procedure

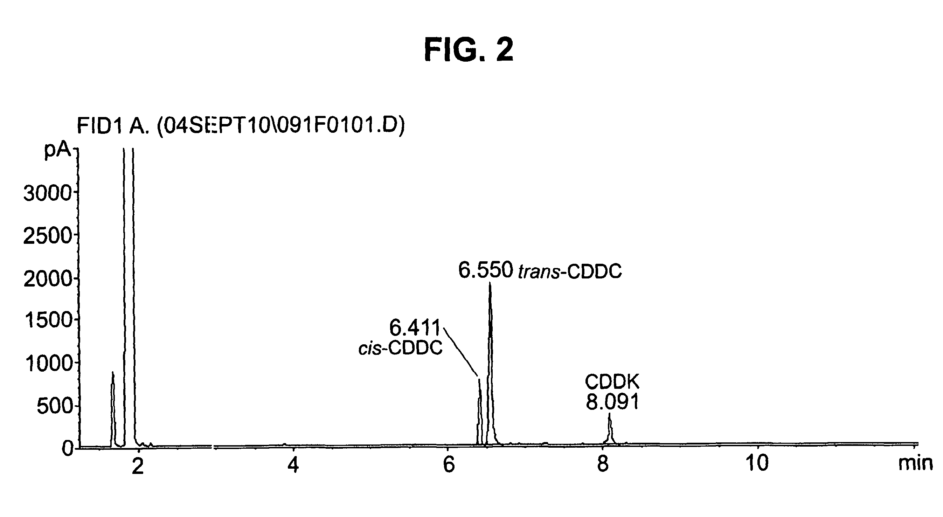

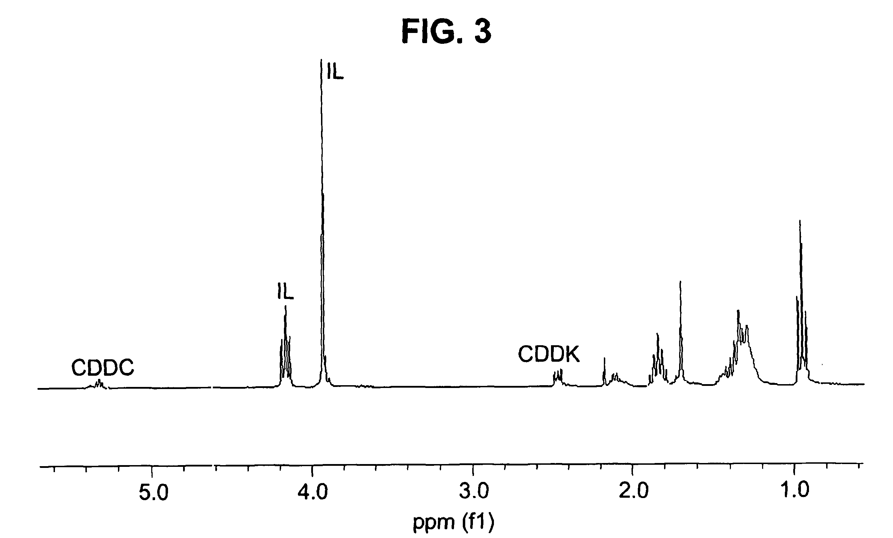

A 20 cm³ autoclave was initially charged with 1 g of cyclododecene (6.01 mmol) and 1 g of ionic liquid, sealed and flushed with argon. Subsequently, N₂O (10 to 30 bar) was injected to the autoclave and the temperature increased to 200 °C. After 12 h reaction, the autoclave was cooled and decompressed. A biphasic system was obtained in this case. The organic layer (top layer) was analysed by means of quantitative GC (see **Figure 2**) while the ionic liquid layer was analysed by ¹H NMR (see **Figure 3**). The different results are summarised in Table 2. The conversion of cyclododecene is based on the GC analysis since only a small amount of cyclododecanone was observed in the ionic liquid layer (the real conversion values should be slightly higher then those depicted in Table 2).

**Table 2**

| Solvent | Pressure N₂O injected (bar) at 20 °C | Temperature (°C) | Maximum pressure in the autoclave | Time (h) | Conversion (%) | CDDK-selectivity (%) |
|---|---|---|---|---|---|---|
| [bmim][NTf₂] | 10 | 200 | 15 | 12 | 4 | ≥ 99 |
| [bmim][NTf₂] | 20 | 200 | 35 | 12 | 10 | ≥ 99 |
| [bmim][NTf₂] | 30 | 200 | 50 | 12 | 16 | ≥ 99 |
| [bmim][NTf₂] | 45 | 200 | 75 | 12 | 24 | 98 |
| [bmim][Ga₂Cl₇] | 30 | 200 | 50 | 12 | 16 | ≥ 99 |
| [bmim][In₂Cl₇] | 30 | 200 | 47 | 12 | 4 | ≥ 99 |

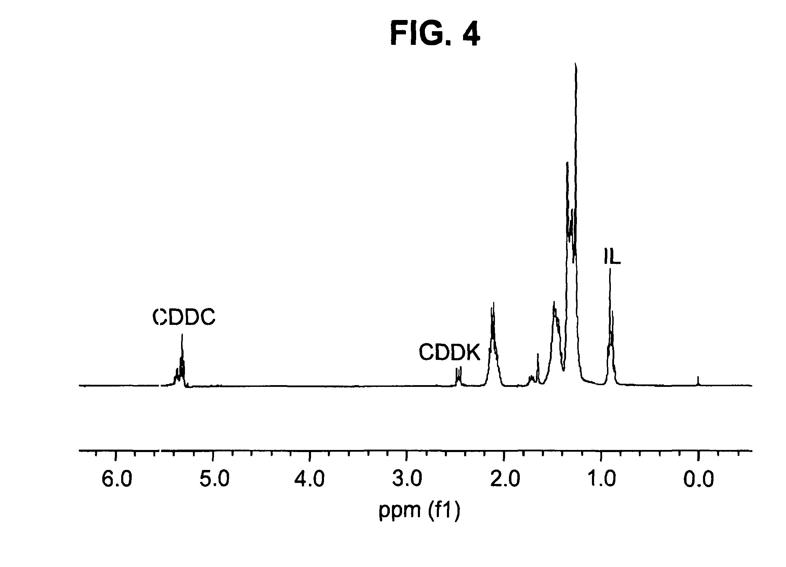

Further reactions were carried out using phosphonium ionic liquids in an analogous manner to the reactions carried out above for [bmim] ionic liquids. The results are shown in Table 3 below and **Figure 4** (NMR analysis).

**Table 3**

| **Solvent** | **Pressure (bar)** | **Temperature (°C)** | **Maximum pressure in the autoclave** | **Time (h)** | **Conversion (%) (¹H NMR)** | **CDDK-selectivity (%)** |
|---|---|---|---|---|---|---|
| P₆₆₆₁₄][NTf₂] | 30 | 200 | 47 | 12 | 40 | |
| [P₆₆₆₁₄][NTf₂] | 45 | 200 | 74 | 12 | 40 | 98 |
| [P₆₆₆₁₄][Ga₂Cl₇] | 30 | 200 | 50 | 12 | Black viscous solution * | |
| [P₆₆₆₁₄][Ga₂Cl₇] | 30 | 200 | 50 | 3 | * | |
| [P₆₆₆₁₄][Ga₂Cl₇] | 30 | 20 | 30 | 3 | * | |
| [P₆₆₆₁₄][Ga₂Cl₇] | 0 | 200 | 0 | 3 | * | |
| [P₆₆₆₁₄][InCl4] | 30 | 200 | 50 | 12 | 35 | |
| [P₆₆₆₁₄][InCl4] | 45 | 200 | 75 | 12 | 50 | 90 |

| | | | | | | |
|---|---|---|---|---|---|---|
| * 1H NMR shows that no CDDC is left in the solution however no formation of CDDK is observed | | | | | | |

### GC method:

Instrument: Agilent 6890N GC
Column: HP-5 (5% phenyl)-methylsiloxane

### Uven program

**Initial temperature: 40 °C**

| Ramps: rate (°C/min) | final temp. | final time (min) |
|---|---|---|
| 15 | 200 | 0.00 |
| 50 | 320 | 2.00 |

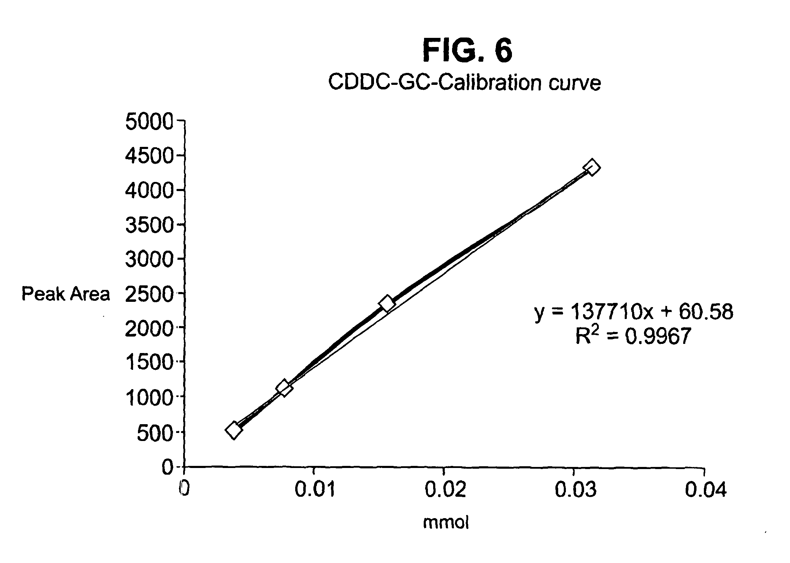

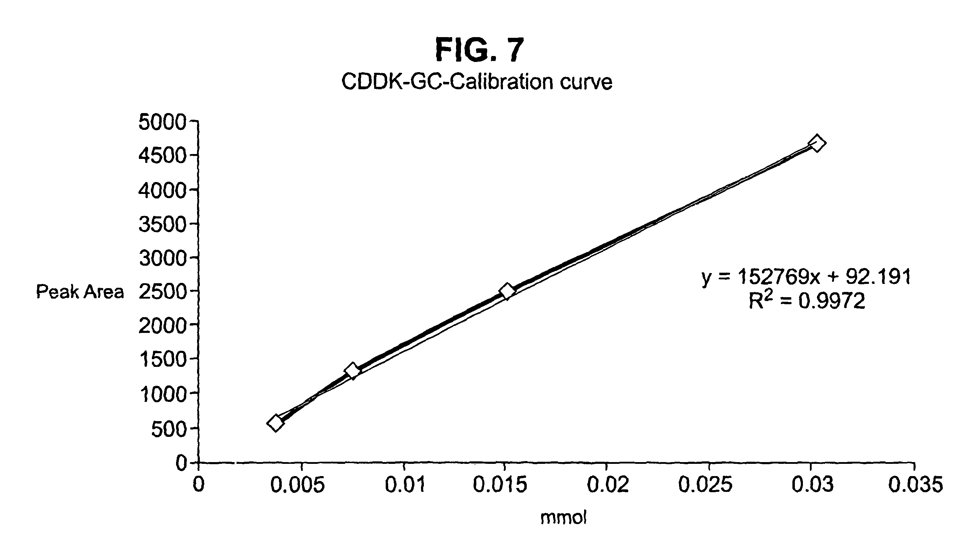

GC calibration curves are shown in **Figures 6** and **7****.**
NMR measurements were carried out on a Bruker Avance DPX, 300 Mhz instrument.

## Claims

1. Use of an M₂O-containing ionic liquid as a reagent in a chemical reaction wherein the N₂O-containing ionic liquid is used as an oxidising agent, and wherein the N2O-containing ionic liquid is obtained via a reaction process comprising the steps:
(a) contacting a reactant with a reagent to produce a product and a mixture of by-products comprising N₂O; and
(b) contacting the mixture of by-products with an ionic liquid to absorb the N₂O to produce an N₂O-containing ionic liquid.

2. Use according to claim 1, wherein the reaction to obtain the N-₂O-containing ionic liquid is an oxidation reaction, the reagent in step (a) is an oxidising agent and the product in step (a) is an oxidised product.

3. Use according to claim 2, wherein the oxidising agent is nitric acid.

4. Use according to claim 2 or 3, wherein the reactant in step (a) is selected from cyclohexanol, cyclohexanone or a mixture of cyclohexanol and cyclohexanone and the oxidised product is adipic acid.

5. Use according to any one of claims 1 to 4, wherein the reactant in step (a) is produced by the hydrogenation of phenol.

6. Use according to claim 5, wherein the phenol is produced by the oxidation of benzene.

7. Use according to any one of claims 1 to 6, wherein the use comprises oxidising benzene to produce phenol.

8. Use according to any one of claims 1 to 6, where in the use comprises the oxidative dehydrogenation of an alcohol to a ketone.

9. Use according to any one of claims 1 to 6, wherein the use comprises the oxidative dehydrogenation of butene to 1,3-butadiene.

10. Use according to any one of claims 1 to 6, wherein the use comprises the oxidation of cyclohexene to cyclohexanol, cyclohexanone or a mixture of cyclohexanol and cyclohexanone.

11. Use according to any one of claims 1 to 6, wherein the use comprises the oxidation of cyclododecene to cyclododecanone.

12. Use according to any one of claims I to 11, wherein the N₂O-containing ionic liquid comprises an imidazolium cation.

13. Use according to any one of claims 1 to 11, wherein the N₂O-containing ionic liquid comprises an N-methyl-N'-butylimidazolium cation.

14. Use according to any one of claims 1 to 11, wherein the N₂O-containing ionic liquid comprises a phosphonium cation.

15. Use according to any one of claims 1 to 11, wherein the N₂O-containing ionic liquid comprises atetradecyltrihexylphosphonium cation.

## Patentansprüche

1. Verwendung einer N₂O-haltigen ionischen Flüssigkeit als Reagens in einer chemischen Reaktion, wobei die N₂O-haltige ionische Flüssigkeit als Oxidationsmittel verwendet wird und wobei die N₂O-haltige ionische Flüssigkeit durch ein Reaktionsverfahren erhalten wird, das folgende Schritte umfasst:
(a) Inberührungbringen eines Reaktanten mit einem Reagens zur Herstellung eines Produkts und eines Gemischs von Nebenprodukten, das N₂O umfasst; und
(b) Inberührungbringen des Gemischs von Nebenprodukten mit einer ionischen Flüssigkeit zur Absorption des N₂O zur Herstellung einer N₂O-haltigen ionischen Flüssigkeit.

2. Verwendung nach Anspruch 1, wobei es sich bei der Reaktion zum Erhalt der N₂O-haltigen ionischen Flüssigkeit um eine Oxidationsreaktion handelt und es sich bei dem Reagens in Schritt (a) um ein Oxidationsmittel handelt und es sich bei dem Produkt in Schritt (a) um ein oxidiertes Produkt handelt.

3. Verwendung nach Anspruch 2, wobei es sich bei dem Oxidationsmittel um Salpetersäure handelt.

4. Verwendung nach Anspruch 2 oder 3, wobei der Reaktant in Schritt (a) aus Cyclohexanol, Cyclohexanon oder einem Gemisch von Cyclohexanol und Cyclohexanon ausgewählt wird und es sich bei dem oxidierten Produkt um Adipinsäure handelt.

5. Verwendung nach einem der Ansprüche 1 bis 4, wobei der Reaktant in Schritt (a) durch Hydrierung von Phenol hergestellt wird.

6. Verwendung nach Anspruch 5, wobei das Phenol durch Oxidation von Benzol hergestellt wird.

7. Verwendung nach einem der Ansprüche 1 bis 6, wobei die Verwendung die Oxidation von Benzol zu Phenol umfasst.

8. Verwendung nach einem der Ansprüche 1 bis 6, wobei die Verwendung die oxidative Dehydrierung eines Alkohols zu einem Keton umfasst.

9. Verwendung nach einem der Ansprüche 1 bis 6, wobei die Verwendung die oxidative Dehydrierung von Buten zu 1,3-Butadien umfasst.

10. Verwendung nach einem der Ansprüche 1 bis 6, wobei die Verwendung die Oxidation von Cyclohexen zu Cyclohexanol, Cyclohexanon oder einem Gemisch von Cyclohexanol und Cyclohexanon umfasst.

11. Verwendung nach einem der Ansprüche 1 bis 6, wobei die Verwendung die Oxidation von Cyclododecen zu Cyclododecanon umfasst.

12. Verwendung nach einem der Ansprüche 1 bis 11, wobei die N₂O-haltige ionische Flüssigkeit ein Imidazoliumkation umfasst.

13. Verwendung nach einem der Ansprüche 1 bis 11, wobei die N₂O-haltige ionische Flüssigkeit ein N-Methyl-N'-butylimidazoliumkation umfasst.

14. Verwendung nach einem der Ansprüche 1 bis 11, wobei die N₂O-haltige ionische Flüssigkeit ein Phosphoniumkation umfasst.

15. Verwendung nach einem der Ansprüche 1 bis 11, wobei die N₂O-haltige ionische Flüssigkeit ein Tetradecyltrihexylphosphoniumkation umfasst.

## Revendications

1. Utilisation d'un liquide ionique contenant du N₂O comme un réactif dans une réaction chimique dans laquelle le liquide ionique contenant du N₂O est utilisé comme un oxydant, et dans laquelle le liquide ionique contenant du N₂O est obtenu par un procédé réactionnel comprenant les étapes :
(a) mettre un réactant en contact avec un réactif pour générer un produit et un mélange de sous-produits comprenant du N₂O ; et
(b) mettre le mélange de sous-produits en contact avec un liquide ionique pour absorber le N₂O pour produire un liquide ionique contenant du N₂O.

2. Utilisation selon la revendication 1, dans laquelle la réaction pour obtenir le liquide ionique contenant du N₂O est une réaction d'oxydation, le réactif à l'étape (a) est un oxydant et le produit à l'étape (a) est un produit oxydé.

3. Utilisation selon la revendication 2, dans laquelle l'oxydant est l'acide nitrique.

4. Utilisation selon la revendication 2 ou 3, dans laquelle le réactant à l'étape (a) est choisi parmi le cyclohexanol, la cyclohexanone ou un mélange de cyclohexanol et cyclohexanone et le produit oxydé est l'acide adipique.

5. Utilisation selon l'une quelconque des revendications 1 à 4, dans laquelle le réactant à l'étape (a) est produit par l'hydrogénation de phénol.

6. Utilisation selon la revendication 5, dans laquelle le phénol est produit par l'oxydation de benzène.

7. Utilisation selon l'une quelconque des revendications 1 à 6, l'utilisation comprenant l'oxydation de benzène pour produire du phénol.

8. Utilisation selon l'une quelconque des revendications 1 à 6, l'utilisation comprenant la déshydrogénation oxydative d'un alcool en une cétone.

9. Utilisation selon l'une quelconque des revendications 1 à 6, l'utilisation comprenant la déshydrogénation oxydative de butène en 1,3-butadiène.

10. Utilisation selon l'une quelconque des revendications 1 à 6, l'utilisation comprenant l'oxydation de cyclohexène en cyclohexanol, cyclohexanone ou un mélange de cyclohexanol et cyclohexanone.

11. Utilisation selon l'une quelconque des revendications 1 à 6, l'utilisation comprenant l'oxydation de cyclododécène en cyclododécanone.

12. Utilisation selon l'une quelconque des revendications 1 à 11, dans laquelle le liquide ionique contenant du N₂O comprend un cation imidazolium.

13. Utilisation selon l'une quelconque des revendications 1 à 11, dans laquelle le liquide ionique contenant du N₂O comprend un cation N-méthyl-N'-butylimidazolium.

14. Utilisation selon l'une quelconque des revendications 1 à 11, dans laquelle le liquide ionique contenant du N₂O comprend un cation phosphonium.

15. Utilisation selon l'une quelconque des revendications 1 à 11, dans laquelle le liquide ionique contenant du N₂O comprend un cation tétradécyltrihexylphosphonium.
